Europäisches Patentamt

European Patent Office

Office européen des brevets

(19)

(11) Publication number: **0 029 715**
B1

(12) **EUROPEAN PATENT SPECIFICATION**

(45) Date of publication of patent specification: **07.03.84**

(21) Application number: **80304169.8**

(22) Date of filing: **20.11.80**

(51) Int. Cl.³: **A 61 K 33/44,**
**C 01 B 31/14, B 01 J 39/24**

(54) **Porous spherical carbonaceous product and production thereof.**

(30) Priority: **22.11.79 JP 151645/79**

(43) Date of publication of application:
**03.06.81 Bulletin 81/22**

(45) Publication of the grant of the patent:
**07.03.84 Bulletin 84/10**

(84) Designated Contracting States:
**DE FR GB IT NL**

(56) References cited:
**DE - A - 2 123 067**
**DE - B - 1 301 978**
**FR - A - 2 413 092**
**GB - A - 763 165**
**US - A - 3 917 806**

(73) Proprietor: **KUREHA KAGAKU KOGYO KABUSHIKI KAISHA**
**9-11 Horidome-cho 1-chome Nihonbashi Chuo-ku Tokyo (JP)**

(72) Inventor: **Endo, Hiroshi**
**4-32-11-305 Kami-Kitazawa Setagaya-ku Tokyo (JP)**
Inventor: **Katoh, Masao**
**2-1-4 Higashi-Koshigaya Koshigaya-shi Saitama-ken (JP)**
Inventor: **Hino, Kuniaki**
**5-31-1 Matsubara Setagaya-ku Tokyo (JP)**

(74) Representative: **Myerscough, Philip Boyd et al, J.A.Kemp & Co. 14, South Square Gray's Inn London, WC1R 5EU (GB)**

Courier Press, Leamington Spa, England.

# O 029 715

## Porous spherical carbonaceous product and production thereof

The present invention relates to a porous and spherical carbonaceous product having acidic, basic, phenolic hydroxyl and carboxyl functional groups and to a method of preparing this product.

In recent years, a method of removing toxic substances within living bodies by a hemodialyzer as an artificial organ has prevailed with increased cases of disease due to troubles in renal and hepatic functions. However, skilled specialists are required for operation of the complicated apparatus of a hemodialyzer. There is a high physical and mental strain for the patient because it is necessary to take the patient's blood out from his body. The method is relatively expensive.

A method of removing endogenous toxins formed due to liver and kidney diseases within the living body by internal administration of an orally administered pharmaceutical substance, for instance oxidized starch, has recently been proposed. However, the proposed medicine is a reactive substance. Although it is able to remove intestinal urea, it cannot remove the essentially endogenous toxins caused by the liver and kidney diseases such as octapamine, betaamino-iso-butyric acid and dimethylamine. It also has a side-reaction of stimulating the intestinal wall. Accordingly, use of such a medicine is not preferable.

On the other hand, in view of the favourable properties of medicinal activated carbon such as its adsorption of various substances and its safety to living bodies, trials for the removal of the endogenous toxins caused by liver and kidney diseases have been carried out using such carbon. However, although the carbon is useful as an antidote which adsorbs and removes gastrointestinal toxic substances taken from the mouth, it has insufficient ability to remove the endogenous toxins in the intestines due to diseases of the liver and kidney. This is because of the presence of substances such as bile acids which coat the surface of active carbon and interfere with the activity of the carbon. In addition, the carbon is apt to cause constipation after oral administration and this constipation can be particularly dangerous to patients suffering from liver or kidney diseases.

It has now been found that these problems can be overcome by using a porous and spherical carbonaceous product containing specified functional groups in specified mutual relationships. The product exhibits an excellent absorptive effect on endogenous toxins caused by liver and kidney diseases, even in the presence within the intestinal tracts of bile acid, while not absorbing digestive enzymes in the gastrointestinal tracts and not causing symptoms of constipation.

The present invention therefore provides a spherical and carbonaceous product having acidic, basic, phenolic hydroxyl and carboxyl functional groups wherein the product contains 0.30 to 1.20 equivalent of the acidic groups/kg of the product, 0.20 to 0.70 equivalent of the basic groups/kg of the product, 0.20 to 0.70 equivalent of the phenolic hydroxyl groups/kg of the product and less than 0.15 equivalent of the carboxyl groups/kg of the product, the ratio of equivalents of said acidic groups to equivalents of said basic groups is from 0.40:1 to 2.5:1 and the value obtained by subtracting the equivalents of said carboxyl groups from the sum of the equivalents of said basic groups and the equivalents of said phenolic hydroxyl groups is greater than 0.60 equivalent/kg.

The product of the invention can be prepared by a method comprising dispersing a mixture of an aromatic hydrocarbon and a heavy hydrocarbon of an atomic ratio of hydrogen to carbon 0.45:1 to 0.8:1 in which the presence of an uneven distribution of anisotropic regions is not observable under a polarization microscope, in hot water containing a surfactant under agitation, thereby shaping said mixture into minute spherical particles; bringing said minute particles into contact with an oxidative gas flow, thereby obtaining minute particles containing 7 to 25% by weight of oxygen; heating the thus obtained minute particles containing 7 to 25% by weight of oxygen at a temperature of 800 to 1000°C in a flow of gas or gaseous mixture reactive to carbon at high temperatures, thereby obtaining a first porous spherical carbonaceous substance; heating said first porous spherical carbonaceous substance at a temperature of 350 to 700°C in an atmosphere containing 0.5 to 20% by volume of oxygen or an oxygen-containing gas; and further heating at a temperature of 800 to 1000°C in an atmosphere inert to carbon.

The porous and spherical carbonaceous product of the present invention has, as functional groups, acidic groups, basic groups, phenolic hydroxyl groups and carboxyl groups in the following mutual relationships:

(1) the ratio of equivalents of the acidic groups to the equivalents of the basic groups is in the range of 0.40:1 to 2.5:1, and

(2) the value obtained by subtracting the equivalents of carboxylic groups from the sum of the equivalents of the basic groups and the phenolic hydroxyl groups is larger than 0.60.

When the amounts of the acidic, basic, phenolic hydroxyl and carboxyl groups are represented respectively by A, B, C and D equivalent/kg of the material (hereinafter referred to as eq/kg), the relationships become as follows:

(1) $A/B = 0.40$ to $2.5$, and

(2) $(B + C) - D = 0.60$.

The product of the present invention contains the above-mentioned functional groups in the amounts shown below:

Acidic groups : 0.30 to 1.20 eq/kg (A eq/kg)

2

O 029 715

Basic groups : 0.20 to 0.70 eq/kg (B eq/kg)
Phenolic hydroxyl groups : 0.20 to 0.70 eq/kg (C eq/kg) and
Carboxyl groups : less than 0.15 eq/kg (D eq/kg).
The amount of the respective functional groups is quantitatively determined as follows:

a) Acidic group (A group)

After adding $10^{-3}$kg (one gram) of a pulverized specimen of the product of the present invention passing through a Taylor standard sieve of 200 mesh to $5 \times 10^{-}m^3$ (50 ml) of an aqueous 0.05 N NaOH solution and shaking the mixture for 48 hours, the mixture is filtered to remove the specimen. The filtrate is titrated to neutrality. The amount of A groups is represented by the amount of NaOH consumed by the specimen expressed as eq/kg of the specimen.

b) Basic group (B group)

After adding $10^{-3}$kg (one gram) of a pulverized specimen of the product of the present invention passing through a Taylor standard sieve of 200 mesh to $5 \times 10^{-5}m^3$ (50 ml) of an aqueous 0.05 N HNI solution and shaking the mixture for 24 hours, the mixture is filtered to remove the specimen. The filtrate is titrated to neutrality. The amount of B groups is represented by the amount of HCl consumed by the specimen expressed as eq/kg of the specimen.

c) Phenolic hydroxyl group (C group)

After adding $10^{-3}$kg (one gram) of a pulverized specimen of the product of the present invention passing through a Taylor standard sieve of 200 mesh to $5 \times 10^{-}m^3$ (50 ml) of an aqueous 0.05 N $Na_2CO_3$ solution and shaking the mixture for 24 hours, the mixture is filtered to remove the specimen. The filtrate is titrated to neutrality. The amount of C groups is represented by the balance obtained by subtracting the amount of $Na_2CO_3$ consumed by the specimen from the amount of acidic groups, expressed as eq/kg of the specimen.

d) Carboxyl group (D group)

After adding $10^{-3}$kg (one gram) of a pulverized specimen of the product of the present invention passing through a Taylor standard sieve of 200 mesh to $5 \times 10^{-5}m^3$ (50 ml) of an aqueous 0.05 N $NaHCO_3$ solution and shaking the mixture for 24 hours, the mixture is filtered to remove the specimen. The filtrate is titrated to neutrality. The amount of D groups is represented by the amount of $NaHCO_3$ consumed by the specimen, expressed as eq/kg of the specimen.

The fact that the specified relationships between the various functional groups is specific to the product of the present invention can be understood from Table 1. In this Table the above-mentioned specified relationships are compared with those of a commercial spherical activated carbon having the same shape.

TABLE 1

| Functional group amounts in eq/kg | Porous and spherical carbonaceous product, the product of the present invention | A commercial spherical activated carbon |
|---|---|---|
| Acidic groups (A) | 0.30 to 1.20 | 0.13 |
| Basic groups (B) | 0.20 to 0.70 | 0.44 |
| Phenolic hydroxyl group (C) | 0.20 to 0.70 | 0.13 |
| Carboxyl group (D) | less than 0.15 | 0.001 |
| A/B | 0.40 to 2.5 | 0.3 |
| (B + C) − D | larger than 0.60 | 0.57 |

The porous and spherical carbonaceous substance according to the present invention preferably consists of porous and spherical particles of $5 \times 10^{-5}$ to $10^{-3}$m (0.05 to 1 mm) in diameter, a specific pore volume for pores with radii of less than $8 \times 10^{-9}$m (80 Å) of $2 \times 10^{-4}$ to $10^{-3}m^3$/kg (0.2 to 1.0 cc/g) and a specific pore volume for pores with radii of $10^{-8}$ to $7.5 \times 10^{-6}$m (100 to 75,000 Å) of $10^{-4}$ to $10^{-3}m^3$/kg (0.1 to 1.0 cc/g). The specific pore volume for pores with radii of less than $8 \times 10^{-9}$m (80 Å) is determined by a conventional gas adsorption method, and that for pores with radii of $10^{-8}$ to $7.5 \times 10^{-6}m^3$ (100 to 75,000 Å) is determined by a mercury porosimeter.

The porous and spherical carbonaceous product according to the present invention can be produced by the following method:

(a) Preparation of a first porous spherical carbonaceous substance as a precursor of the product of the present invention.

3

A heavy hydrocarbon of a ratio of H/C of 0.45 to 0.80 and of a flow point of 100 to 300°C, in which the presence of unevenly distributed anisotropic regions is not observed under a polarization microscope, is used as the starting material. A mixture of this heavy hydrocarbon and an aromatic hydrocarbon such as benzene and naphthalene is dispersed in hot water, preferably at a temperature of 100 to 180°C, containing a surfactant while stirring the mixture of the hydrocarbons and hot water to shape minute particles of the mixture of hydrocarbons. After cooling the dispersion to room temperature, the thus formed and solidified minute particles are separated from the greater part of the aqueous phase by filtration. The aromatic hydrocarbon contained in the minute particles is removed by extraction with an organic solvent such as hexane or methanol. The thus extracted particles are brought into contact with a flow of an oxidative gas, for instance an oxygen flow, to effect oxidation of the minute particles. The content of oxygen in the resulting particles is 7 to 25% by weight. Then, the thus partially oxidized minute particles are heated in a flow of gas having a reactivity to carbon at high temperatures, such as steam and gaseous carbon dioxide, at a temperature of 800 to 1000°C to convert the particles into a first porous and spherical carbonaceous substance.

(B) Production of the present product

The first porous and spherical carbonaceous substance obtained above is heated at a temperature of 350 to 700°C, preferably at 400 to 600°C in an atmosphere containing 0.5 to 20% by volume, preferably 3 to 10% by volume, of oxygen, and is then further heated at a temperature of 800 to 1000°C in an atmosphere of a gas inert to carbon, for instance nitrogen, argon or helium or of a mixture of such inert gases to obtain the porous and spherical carbonaceous substance of the present invention. The atmosphere containing oxygen can be formed by using oxygen, nitrogen oxides or air as the oxygen source.

The series of heat-treatments described above confers on the spherical carbonaceous substance a balance between the adsorbency of acidic and basic substances as well as an adsorbency to amphoteric substances. In addition, the heat-treatments confer on the porous and spherical carbonaceous substance the selective adsorbing sites expressed by "the ratio of equivalents of the acidic groups to equivalents of the basic groups" and "the value obtained by subtracting the equivalents of the carboxylic groups from the sum of the equivalents of the basic groups and the phenolic hydroxyl groups, $(B + C) - D$",

Heat-treatment in an atmosphere containing oxygen only may improve the adsorptive ability to basic substances such as amine. However, the adsorptive ability to the amphoteric substance can not be improved in this way.

The spherical carbonaceous substance of the present invention exhibits a specific adsorptive ability to endogenous toxins within the living bodies, as will be described later, which could not be presumed from the adsorptive ability of conventional activated carbon. Although the adsorption mechanism has not yet been elucidated, it is presumed to be due to the selectivity of the adsorption sites caused by the specified relationships among the various functional groups on the internal surface of the porous and spherical carbonaceous substance and the synergistic function of the charged potential on the outer surface and of the minute physical structure of the product of the invention.

The product of the present invention can be used in treating liver and/or kidney diseases, especially diseases aggravated and/or caused by endogenous toxin(s) in the intestines of a human or mammal. It is possible for the present product to exhibit an adsorptive ability which has hitherto not been demonstrated in activated carbon including conventional carbons for pharmaceutical use. The present product exhibits its adsorptive ability to endogenous toxins even in the presence of bile acids. In other words, the present product effectively adsorbs octopamine and gammaminobutyric acid which are the casual substance of hepatic encephalopathy, endogenous toxins and their precursors in kidney diseases, for example water-soluble basic and amphoteric substances such as dimethylamino beta-amino-isobutyric acid, asparatic acid and arginine. In addition, the present product adsorbs only a little of the digestive enzymes within intestines, which preferably ought not to be removed. Moreover, it does not cause constipation.

Accordingly, the present product can work well in the treatment of tremor and cerebral disorders due to liver diseases and in the treatment of metabolic abnormality and functional abnormality, and in the improvement of light renal disorder before hemodialysis in kidney diseases and of the conditions during hemodialysis. In addition, it can work well in the treatment of other diseases, for example pshchosis due to noxious substances within the living body.

Where the present product is applied as a medicine for treating diseases of the liver and/or kidney, its dose depends on the patient concerned, such as whether the patient is human or another mammal, the age of the patient, and the particular condition of the patient. In humans, the usual oral dose is $10^-$ to $5 \times 10^{-3}$kg (1 to 5g)/day taken in 3 or 4 portions during the day. However, the dose is dependent on the factors explained above and may be increased or decreased as appropriate.

The present product may take any one of several shapes and forms when it is to be administered as a medicine. The product may be in the form of particles, granules, tablets, sugar-coated tablets, capsules or a suspension. In the case where it is administered as capsules, gelatin and/or other substances which dissolve within the intestines can be used to encapsulate the product. In the cases of using tablets, it is necessary for the tablets to disintegrate into the orginial minute spherical particles of the

0 029 715

product of the invention within the body of a patient. In addition, the present product may be combined with an electrolyte-controlling agent such as aluminum gel or KAYEXALATE (produced by Winthrop Lab., USA) and administered as a combined medicine.

The product of the present invention has an extremely low toxicity to mammals as can be seen below:

A. Acute toxicity test in mammals:

Each of the porous and spherical carbonaceous substances of the present invention prepared in Examples 1, 2 and 3 below was administered via a stomach tube to a respective group of male and feamlae JCL—SD rats (average body weight of $8.78 \times 10^{-2} \pm 4.3 \times 10^{-3}$ kg (87.8 $\pm$ 4.3 g) for female rats, and of $9.78 \times 10^{-2} \pm 4.0 \times 10^{-3}$ kg (97.8 $\pm$ 4.0 g) for male rats). Each group consisted of 10 animals of one sex. After one week of observations, no death was recorded. When autopsies were carried out after the end of the period of observation, no abnormal findings were obtained from external or from internal observation on several organs as well as no noticeable symptons of intoxication due to the administration. The respective $LD_{50}$ values of the products of the invention that were tested are shown in Table 2.

TABLE 2

$LD_{50}$ of the Products of Invention

| Specimen | Route of Administration | Sex of rats | $LD_{50}$ $(10^{-6}$kg/kg) (mg/kg) |
|---|---|---|---|
| Product of | p.o. | Female | over 18,000 |
| Example 1 | | Male | over 18,000 |
| Product of | p.o. | Female | over 18,000 |
| Example 2 | | Male | over 16,000 |
| Product of | p.o. | Female | over 18,000 |
| Example 3 | | Male | over 16,000 |

B. Sub-acute toxicity test on mammals:

Five kinds of different solid diets were given for one month to 10 groups of JCL—SD rats of age 4 weeks. Each group consisted of either 10 males or 10 females and each diet was fed to 1 group of males and 1 group of females. The five kinds of diet respectively contained 0, 1, 2, 5 and 10% by weight of the product of Example 3 below. The diet and water were taken *ad lib*. During the period of administration of the diet, the amounts of water and the diet taken by the rats and the body weight of the rats were measured, and their behaviour was observed.

After the period of administration was over, blood specimens were collected from the rats and then the rats were sacrificed to be autopsied. The average amount of diets were taken by the rats during the period of adminsitration was as follows, respectively corresponding to the content of the present product of 1, 2, 5 and 10% by weight: 1100, 2300, 5700 and 11400 $10^{-6}$kg/kg (mg/kg) body weight of female rat, and 1400, 2800, 7000 and 14000 $10^{-6}$kg/kg (mg/kg) body weight of male rat.

Although a slight suppression of body weight gain was observed in the middle stages of the test on the group of male rats which took the diet of the highest content of the product of the invention, body weight recovered afterwards. Except for this finding, no noticeable abnormal findings were obtained for body weight gain, the general conditions, blood examination, urinalysis, autopsy, weight of organs and pathohistological examination of various tissues as well as symptoms of intoxication.

The following Examples illustrate the present invention:

Example 1

$3 \times 10^{-1}$kg (three hundred grams) of a heavy hydrocarbon of H/C of 0.55, a flow point of 220°C and not having localized presence of anisotropic region under a polarization microscope, and $10^{-1}$kg (100 g) of naphthalene was introduced into an autoclave provided with a stirrer. The mixture was further mixed well at a temperature of 180°C. Into the thus obtained liquid mixture, 1.2 kg (1200 g) of an aqueous 0.5% solution of polyvinyl alcohol (degree of saponification of 88%) were added. Then, the mixture was vigorously stirred at a temperature while stirring to form a dispersion of spherical particles of from $7 \times 10^{-5}$ to $1.2 \times 10^{-3}$m (0.07 to 1.2 mm) in diameter. After separating the greater part of the water from the spherical particles, the particles were treated with hexane in an extractor to remove

5

naphthalene contained in the particles by extraction, and dried by air flow. The resulting particles were heated to 300°C at a rate of temperature rise of 25°C/hour by a flow of heated air in a fluidized bed system and further heated for 2 hours at a fixed temperature of 300°C. Spherical particles containing 14% by weight of oxygen were obtained. These particles were heated to a temperature of 900°C by steam in a fluidized bed system. The particles were heated for two hours at this temperature to obtain the porous and spherical carbonaceous particles which are the precursor of the product of the present invention.

The precursor particles were heated to a temperature of 600°C in an atmosphere containing 3% by volume of oxygen, and heated for a further 3 hours in the same atmosphere. Then, the precursor particles were further heated to a temperature of 950°C in an atmosphere of nitrogen and kept at that temperature for 30 min in an atmosphere of nitrogen to obtain the product of the present invention. The properties and the content of the various functional groups in this product are shown in Table 3.

Example 2

$3 \times 10^{-1}$ kg (three hundred grams) of a heavy hydrocarbon of H/C of 0.65 and of a flow point of 210°C and $10^{-1}$ kg (100 g) of naphthalene were introduced into an autoclave provided with a stirrer and co-melted by heating to a temperature of 180°C while stirring. 1.2 kg (1200 g) of an aqueous 0.5% solution of polyvinyl alcohol of a degree of saponification of 88% was added to the liquid mixture thus-obtained. The resulting mixture was agitated vigorously at a temperature of 130°C for 30 min. On cooling the mixture to room temperature while stirring, an aqueous dispersion of spherical particles of from $10^{-4}$ to $1.3 \times 10^{-3}$ m (0.1 to 1.3 mm) in diameter was obtained. After removing a greater part of the water from the spherical particles, the particles were treated with hexane in an extractor to remove naphthalene from the particles by extraction. The particles from which naphthalene had been removed were dried by air flow, and heated to a temperature of 300°C by heated air in a fluidized bed system at a rate of temperature rise of 25°C/hour, and then further heated for 2 hours at 300°C. Spherical particles containing 20% by weight of oxygen were obtained. These particles were heated to a temperature of 900°C in steam by fluidized bed system and kept at 900°C for 4 hours in steam to be converted into a porous and spherical carbonaceous substance which is the precursor of the present product. Then, the precursor particles were kept at a temperature of 450°C for 4 hours in an atmosphere containing 10% by volume of oxygen. The particles were further heated to a temperature of 800°C in an atmosphere of nitrogen and kept at 800°C for 30 min in this atmosphere to obtain the present product. The adsorbing property of and the amount of the various functional groups in the product are shown in Table 3.

Example 3

The spherical particles containing 14% by weight of oxygen which had been obtained in the course of Example 1 were heated to a temperature of 900°C in steam by using a fluidized bed system and kept at 900°C for 2 hours in steam to obtain a porous and spherical carbonaceous substance as a precursor of the present product. The precursor particles were kept at a temperature of 550°C in an atmosphere containing 3% by volume of oxygen for 5 hours. The particles were then heated to a temperature of 900°C in an atmosphere of nitrogen and kept at 900°C for 30 min in this atmosphere to obtain the present product. The adsorbing property of and the amount of the various functional groups in the product are shown in Table 3.

Comparative Example

The spherical particles containing 14% by weight of oxygen which had been obtained in the course of Example 1 were heated to 900°C in steam by a fluidized bed system and kept at 900°C for a further 2 hours to obtain a porous spherical carbonaceous substance. This substance was further heated at a temperature of 550°C in an atmosphere containing 3% by volume of oxygen for 5 hours to obtain a porous and spherical carbonaceous substance. The adsorbing property of and the amount of the various functional groups in this latter substance are shown in Table 3.

Example 4

The present Example is concerned with the results of a determination of the adsorptive property and the content of the specified functional groups of the porous and spherical carbonaceous substances produced in Example 1 to 3 and in the Comparative Example, and of a commercial spherical activated carbon for use in hemoperfusion. The adsorptive property was determined on beta-amino-iso-butyric acid, gammaaminobutyric acid, dimethylamine and octopamine in the presence of bile acid *in vitro*. The results are shown in Table 3. The amount of adorbed substance is expressed by the adsorbed amount of (kg/1000 kg) (mg/g) when the specimen was put into an aqueous solution of the above-mentioned substance at a concentration of 50 kg/m³ (5 mg/dl) in the presence of bile acids of a concentration of 0.5% by weight in the aqueous solution.

6

## TABLE 3

Adsorptive Property and Content of Specified Functional
Groups of Porous and Spherical Carbonaceous Product

| | Porous and Spherical Carbonaceous Product | | | | |
| --- | --- | --- | --- | --- | --- |
| | Present Product | | | Comparative Instances | |
| | Example 1 | Example 2 | Example 3 | Comparative Example | Commercial Spherical Activated Carbon |
| Adsorptive Property (kg/100 kg) to | | | | | |
| beta-amino-isobutyric acid | 4.5 | 3.9 | 5.0 | less than 0.5 | 0.5 |
| gamma-amino-butyric acid | 3.0 | 2.7 | 4.5 | less than 0.5 | 0.6 |
| dimethylamine | 13.0 | 12.0 | 13.0 | 9.0 | 3.8 |
| octopamine | 100 | 98 | 105 | 100 | 50 |
| Amount of Functional Groups (eq/kg) | | | | | |
| Acidic group (A) | 0.51 | 0.75 | 0.60 | 1.36 | 0.13 |
| Basic group (B) | 0.42 | 0.33 | 0.39 | 0.14 | 0.44 |
| Phenolic Hydroxyl group (C) | 0.40 | 0.49 | 0.44 | 0.76 | 0.13 |
| Carboxyl group (D) | 0.03 | 0.06 | 0.04 | 0.32 | 0.001 |
| A/B | 1.21 | 2.27 | 1.54 | 9.71 | 0.3 |
| (B + C) − D (eq/kg) | 0.79 | 0.76 | 0.79 | 0.58 | 0.57 |

0 029 715

# 0 029 715

As is seen in Table 3, it is clear that the adsorptive property of the present product, that is the porous and spherical carbonaceous substance of the present invention having the specified functional groups in the specified mutual relationships, is far superior to that of the product of the Comparative Example or the commercial spherical activated carbon hitherto used for hemoperfusion in the treatments of diseases of the liver and kidney.

Example 5

The present Example shows the efficacy of treatment with the present product of experimental animals suffering from an experimental renal failure.

Three groups of female JCL—SAD rats of body weight of $1.8 \times 10^{-1}$ to $2.2 \times 10^{-1}$ kg (180 to 220 g), each group consisting of 10 animals) were incised transversally of their abdomen under anaesthesia. The renal hilums of one of the kidneys of each animal was ligated. After suturing the incised part and naturalizing the rat for one week, the same operation was carried out on the other kidney to prepare the groups of rats suffering from experimental renal failure. While giving diet and water taken *ad lib*, the porous and spherical carbonaceous substance of the invention prepared in Example 3 was forcibly administered to the rats of a first group orally by a stomach tube at a daily dose of 5 kg/1000 kg (500 mg/kg), and the product of the Comparative Example was administered to the rats of a second group in the same manner. The rats of a third group were administered nothing. The state of the rats of the three groups was observed to find the survival period (days) after the second operation. The results are shown in Table 4. As is seen in Table 4, the survival period of the group to which the product of Example 3 was administered was clearly longer than those of the groups of rats which were treated with the product of the Comparative Example and with nothing, respectively.

8

TABLE 4

Survival Period of Rats

Unit: day

| Group | Survival Period (average) |
|---|---|
| No. 1 administered with the product of Example 3 | 4.2 |
| No. 2 administered with the product of the Comparative Example | 2.8 |
| No. 3 nothing administered | 2.0 |

Example 6

This Example is concerned with the treatment of a human patient with renal failure with the product of the present invention.

The patient, a man of age 44, suffering from chronic renal failure showed a level of creatinine of 20 to 40 $kg/m^3$ (2 to 4 mg/dl) for a long time, afterwards a raised level of creatinine of 80 $kg/m^3$ (8 mg/dl) after one month and then a still further raised level of creatinine of 110 $kg/m^3$ (11 mg/dl) after two months accompanied by the subjective symptoms of loss of appetite and tiredness. The product prepared in Example 3 was then administered to the patient in the form of gelatin capsules by internal administration at a daily dose of $3 \times 10^{-3}$ to $4 \times 10^{-3}kg$ (3 to 4 g) for six months.

The level of creatinine of the patient began to show a reduction 2 weeks after the commencement of the treatment and stabilized at 80 $kg/m^3$ to 90 $kg/m^3$ 8 to 9 mg/dl) together with the substantial disappearance of his subjective symptoms.

Example 7

This Example is concerned with the treatment of a human patient with liver disease.

A woman of age 72 suffering from a liver disease, showing a persistent concentration of ammonia in her blood of $2.5 \times 10^{-3}$ to $3.0 \times 10^{-3}kg/m^3$ (250 to 300 micrograms/dl) and having disturbances of consciousness once a month was treated by internal administration of the product of the invention produced in Example 3 at a daily dose of $5 \times 10^{-3}kg$ (5 g) encapsulated in gelatin capsules for 3 months. During the period of treatment, the disturbances of consciousness never occurred and the concentration of ammonia in her blood reduced to $1.5 \times 10^{-3}kg/m^3$ (150 micrograms/dl). After finishing the treatment, a light disturbance of consciousness attacked her after 10 days and the concentration of ammonia in the her blood had increased to $2 \times 10^{-3}kg/m^3$ (200 micrograms/dl). During the period of treatment, the values of examination of her blood and biochemical examination of her blood showed no abnormality.

**Claims**

1. A porous and spherical carbonaceous product having acidic, basic, phenolic hydroxyl and carboxyl functional groups characterised in that the product contains 0.30 to 1.20 equivalent of the acidic groups/kg of the product, 0.20 to 0.70 equivalent of the basic groups/kg of the product, 0.20 to 0.70 equivalent of the phenolic hydroxyl groups/kg of the product and less than 0.15 equivalent of the carboxyl groups/kg of the product, the ratio of equivalents of said acidic groups to equivalents of said basic groups is from 0.40:1 to 2.5:1 and the value obtained by subtracting the equivalent of said carboxyl groups from the sum of the equivalents of said basic groups and the equivalents of said phenolic hydroxyl groups is greater than 0.60 equivalent/kg.

2. A porous and spherical carbonaceous product according to claim 1, wherein said product consists of particles $5 \times 10^{-5}$ to $10^{-3}m$ in diameter.

3. A porous and spherical carbonaceous product according to claim 1 or 2 for use in treating a disease aggravated and/or caused by endogenous toxin(s) in the intestines of a mammal.

4. A method of producing a porous and spherical carbonaceous product as claimed in any one of the preceding claims, characterised by dispersing a mixture of an aromatic hydrocarbon and a heavy hydrocarbon of an atomic ratio of hydrogen to carbon of 0.45:1 to 0.8:1 in which the presence of an uneven distribution of anisotropic regions is not observable under a polarization microscope, in hot water containing a surfactant under agitation, thereby shaping said mixture into minute spherical particles; bringing said minute particles into contact with an oxidative gas flow, thereby obtaining minute particles containing 7 to 25% by weight of oxygen; heating the thus obtained minute particles containing 7 to 25% by weight of oxygen at a temperature of 800 to 1000°C in a flow of gas or gaseous mixture reactive to carbon at high temperatures, thereby obtaining a first porous spherical carbonaceous substance; heating said first porous spherical carbonaceous substance at a temperature of 350° to 700°C in an atmosphere containing 0.5 to 20% by volume of oxygen or an oxygen-containing gas; and further heating at a temperature of 800 to 1000°C in an atmosphere inert to carbon.

## Revendications

1. Produit carboné sphérique et poreux possédant des groupes fonctionnels acide, basique, hydroxyle phénolique et carboxyle caractérisé en ce que le produit contient 0,30 à 1,20 equivalent des groupes acide/kg du produit, 0,20 à 0,70 équivalent des groupes base/kg du produit, 0,20 à 0,70 équivalent des groupes hydroxyle phénolique/kg du produit et moins de 0,15 équivalent des groupes carboxyle/kg du produit, le rapport d'équivalents desdits groupes acide aux équivalents desdits groups basique est de 0,40:1 à 2,5:1 et la valeur obtenue en soustrayant les équivalents desdits groupes carboxyle de la somme des déquivalents desdits groupes base et des équivalents desdits groupes hydroxyle phénolique est supérieure à 0,60 équivalent/kg.

2. Produit carboné sphérique et poreux selon la revendication 1, dans lequel ledit produit consiste en particules de $5 \times 10^{-5}$ à $10^{-3}$ m de diamètre.

3. Produit carboné sphérique et poreux selon la revendication 1 ou 2 pour utilisation dans le traitement d'une maladie aggravée et/ou causée par toxine(s) endogène(s) dans les intestins d'un mammifère.

4. Procédé pour fabriquer un produit carboné sphérique et poreux comme revendiqué dans l'une quelconque des précédentes revendications, caractérisé en ce qu'on disperse un mélange d'un hydrocarbure aromatique et d'un hydrocarbure lourd d'un rapport atomique de l'hydrogène au carbone de 0,45:1 à 0,8:1 dans lequel la présence d'une distribution irrégulière de régions anisotropes n'est pas observable sous un microscope polarisant, dans l'eau chaude contenant un tensioactif sous agitation, façonnant de ce fait ledit mélange en particules sphériques minuscules; on amène lesdites particules en contact avec un courant de gaz oxydant, obtenant de ce fait des particules minuscules contenant 7 à 25% en poids d'oxygène; on chauffe les particules minuscules ainsi obtenues contenant 7 à 25% en poids d'oxygène à une température de 800 à 1000°C dans un courant d'un gaz ou mélange gazeux réactif vis-à-vis du carbone à de hautes températures, obtenant ainsi une première substance carbonée sphérique poreuse; on chauffe ladite première substance carbonée sphérique poreuse à une température de 350 à 700°C dans une atmosphère contenant 0,5 à 20% en volume d'oxygène ou d'un gaz contenant de l'oxygène; et on continue à chauffer à une température de 800 à 1000°C dans une atmosphère inerte vis-à-vis du carbone.

## Patentansprüche

1. Poröses, kugelförmiges Kohlenstoffprodukt mit sauren, basischen, phenolischen Hydroxyl- und Carboxylfunktionellen Gruppen, dadurch gekennzeichnet, daß das Produkt 0,30 bis 1,20 Äquivalente der sauren Gruppen/kg des Produkts, 0,20 bis 0,70 Äquivalente der basischen Gruppen/kg des Produkts, 0,20 bis 0,70 Äquivalente der phenolischen Hydroxylgruppen/kg des Produkts und weniger als 0,15 Äquivalente der Carboxylgruppen/kg des Produkts enthält, wobei das Verhältnis der Äquivalente der sauren Gruppen zu den Äquivalenten der basischen Gruppen von 0,40:1 bis 2,5:1 beträgt, und der Wert, der durch Substraktion der Äquivalente der Carboxylgruppen von der Summe der Äquivalente der basischen Gruppen und der Äquivalente der phenolischen Hydroxylgruppen erhalten wird, größer als 0,60 Äquivalente/kg ist.

2. Poröses, kugelförmiges Kohlenstoffprodukt nach Anspruch 1, bei dem dieses Produkt aus Teilchen mit einem Durchmesser von $5 \times 10^{-5}$ bis $10^{-3}$ m besteht.

3. Poröses, kugelförmiges Kohlenstoffprodukt nach Anspruch 1 oder 2, zur Verwendung bei der Behandlung einer Erkrankung, die durch ein oder mehrere endogene Toxine im Darmtrakt eines Säugetiers verursacht wird.

4. Verfahren zur Hestellung eines porösen, kugelförmigen Kohlenstoffprodukts nach einem der vorangehenden Ansprüche, gekennzeichnet durch Dispergieren einer Mischung aus einem aromatischen Kohlenwasserstoff und einem schweren Kohlenwasserstoff mit einem atomaren Verhältnis von Wasserstoff zu Kohlenstoff von 0,45:1 bis 0,8:1, bei dem unter einem Polarisations-Mikroskop kein Auftreten einer ungleichmäßigen Verteilung von anisotropen Bereichen feststellbar ist, in heißem Wasser, das ein grenzflächenaktives Mittel enthält, wobei gerührt wird, wodurch aus dieser Mischung sehr kleine kugelförmige Teilchen gebildet werden; Inkontaktbringen dieser kleinen Teilchen mit einem oxidierenden Gasstrom, wodurch kleine Teilchen erhalten werden, die von 7 bis 25 Gew.-% Sauerstoff enthalten; Erhitzen der auf diese Weise erhaltenen kleinen Teilchen, die 7 bis 25 Gew.-% Sauerstoff enthalten, bei einer Temperatur von 800 bis 1000°C in einem Strom eines Gases oder einer gas-

**0 029 715**

förmigen Mischung, die bei hohen Temperaturen mit Kohlenstoff reagiert, wodurch eine erste poröse kugelförmige Kohlenstoffstubstanz erhalten wird; Erhitzen dieser ersten porösen kugelförmigen Kohlenstoffsubstanz auf eine Temperatur von 350 bis 700°C in einer Atmosphäre, die 0,5 bis 20 Vol.-% Sauerstoff oder eines sauerstoffhaltigen Gases enthält; und weiteres Erhitzen bei einer Temperatur von 800 vis 1000°C in einer gegenüber Kohlenstoff inerten Atmosphäre.